# EUROPEAN PATENT APPLICATION

(11) **EP 1 155 623 A1**
(43) Date of publication of application: **21.11.2001**
(21) Application number: 00870110.4
(22) Date of filing: 18.05.2000
(51) Int. Cl.: A23K 1/16, A23K 1/18, A61K 35/84, A61P 1/12

(54) **Growth promoter for animals**

(71) Applicant: N.V. Seghers Nutrition Sciences S.A., 9031 Drongen (BE)
(72) Inventor: Molly, Koen, 9870 Zulte-Olsene (BE); Bruggeman, Geert, 8310 St. Kruis (Brugge) (BE)
(74) Representative: Brants, Johan Philippe Emile

(57) **Abstract**

The invention relates to a growth promoter suitable for an animal consisting essentially of a fungi, an extract, a derivative or a mixture thereof, wherein said fungi is chosen from a genera within the Basidiomycetes and to an animal feed comprising 0,01 to 20 weight% of a growth promoter and the use of an animal feed as poultry or pig feed, in particular in their early lifestage.

The invention further relates to a method for the improvement of weight gain and/or for reducing the feed conversion and/or for the improvement of the feed value and/or health and well-being of the animal by providing animals with a fungi or an extract, derivative or a mixture thereof resulting in a sequential action of the specific enumeration of enteric pathogens prior to their specific excretion.

## Description

### Technical field

The present invention relates to a growth promoter suitable for animals, which growth promoter is useful as an animal feed additive having as primary objects the improvement of the microbial ecosystem in the gastro-intestinal tract resulting in an optimalisation of the feed conversion ratio.

The present invention discloses further a process for improving the efficiency of feed utilisation and/or for promoting the growth of animals in which an animal is fed a diet which comprises said growth promoter.

### Background of the invention

In modern animal production systems, the equilibrium between the intestinal flora and host animal is a delicate one, and disturbance of this equilibrium (by e.g. bacterial infection) has a negative impact on the overall performance of the animals (Eckel, 1999). Knowledge about the problems of intestinal microbial infection in livestock opens the door to completely new ways of influencing the bio-regulatory processes through feed additives, reducing the frequency of diarrhoea and even losses, by stabilising the intestinal flora. In the past, this disturbance of the microbial ecosystem in the gastro-intestinal tract of an animal was partially solved by supplying feeds containing antibiotics as growth promoter.

But today, 50 years since the discovery of the traditional antimicrobials (e.g. penicillin), a lot of bacteria are now resistant to one and, in many cases, to multiple antimicrobials (Guillot, 1989). This resistance is proving fatal for thousands of people each year and results in high medical and heavy economic costs (Barton, 1998). The problem of antimicrobial resistance is global, but is partially caused by the world-wide application of antimicrobials in animal nutrition. Addition of those known antimicrobials to feed formulations results in better performance i.e. decreased feed conversions and higher growth rates (Dupont and Steele, 1987; Prescott, 1997; Aarestrup, 1999). For some countries, this led already to a prohibition of almost all antimicrobials, usable as growth promoters in feed formulations (Muirhead, 1998; Ross, 1999).

The problem with most traditional antimicrobials (or some other growth promoters) in use today is that they attack bacteria at the intracellular level (Guillot, 1989). That is, they inhibit key enzymes in the synthesis of compounds used to build up the cell. Whenever this approach is used, bacteria can develop mutations of the enzymes involved or can develop mechanisms to rapidly pump the antimicrobial out of the cell. Alternatively, they can develop enzymes, which directly degrade the antimicrobial (e.g. (β-lactamase) (Neu et al., 1980; Chirica et al., 1998). By plasmid transfer (via microbial conjugation), resistance can be rapidly transferred from one microbial cell to another (expansion of resistance) (Finland, 1971; Hedges and Jacob, 1974; Thompson, 1986; Hamilton, 1994).

Since the world-wide negative response on the use of the traditional antimicrobials (as growth promoters) in animal feeds, research is performed to search for new types of antimicrobials or growth promoters (Mazza, 1998). During research for alternative (natural) antimicrobials or growth promoters, attention is nowadays mainly focussed on the use of several (organic) acids (Eckel, 1997; Liang, 1997; Radecki et al., 1988), new active probiotics (Chiquette and Banchaar, 1998; Garriga et al., 1998; Tannock, 1999), prebiotics (Olsen, 1996; Bower et al., 1998; Brown et al., 1998; Iji and Tivey, 1998; Houdijk et al., 1999), some plant- (onions and garlic) and herb extracts (essential oils) (De Koning and Hongbiao, 1999; Nielsen, 1999).

The main object of the present invention is to provide a growth promoter of natural origin, which does not present the above cited drawbacks.

### Description of the main characteristics of the invention

In a first aspect the invention provides therefor a growth promoter for an animal consisting essentially of a fungus, an extract, derivative or a mixture thereof, said fungus is chosen from a genera within the Basidiomycetes.

The Basidiomycetes for which this invention is applicable include a wide variety of fungi that taxologically belong to the "Basidiomycota", but most preferred for use in this invention are the fungi belonging to the order Agaricales or Aphyllopharales of Homobasidiae, such as for example Armillariella mellea (Fr.) Karst, Tricholoma matsutake (S. Ito et Imai) Sing., Lentinus edodes, (Berk.) Sing.or Shiitake, Coriolus versicolor (Fr.) Quel., Grifola gigantea (Fr.) Pilat, Favolus Arcularius (Fr.) Ames, Ganoderma (Reishi), etc. The taxological nomenclature of the basidiomycetes is based on "Coloured Illustrations of Fungi of Japan" by ROKUYA IMAZEKI and TSUGUO HONGO. Said genera have similar healing effects and have a similar morphological structure.

According to the invention, these fungi can be used in all forms, such as their natural crude form, dried form, as a extract, a derivative or any other form.

Preferably a growth promoter according to the invention is chosen from the genera Lentinus (Shiitake), Ganoderma (Reishi), or Grifola (Maitake). In a more prefered embodiment the fungus is Lentinus edodes, also commonly known as the Shiitake.

The invention provides in a second aspect an animal feed comprising 0,01 to 20 weight% of the above defined growth promoter.

In a third aspect the invention is directed to the use of the animal feed according to the second aspect of the invention as a poultry or a mammal feed, in particular for chicken or pig. The feed according to the invention is of a particular interest for animals in their early lifestage.

In a fourth aspect, the invention is related to a method for the improvement of weight gain and/or for reducing the feed conversion and/or for the improvement of the feed value and/or health and well-being of the animal by providing animals with a fungi or a an extract, derivative or mixture thereof as a growth promoter resulting in a sequential action of the specific enumeration of enteric pathogens prior to their specific excretion.

According to the invention the most preferred fungus is the Lentinus edodes (Shiitake) mushroom.

The Lentinus edodes is a cultured edible fungus, which is very popular in Asia (Chang, 1993). The curative characteristics of Lentinus edodes are studied for humans and are well known since long time (Mizuno, 1995). It was already stated by Wu-Rui in 1620 that "Shiitake accelerates vital energy, wards of hunger, cures colds and defeats body fluid energy". The Lentinus edodes was treated as an elixir of life for humans, however without any scientific verification. In the 1970's, β-D-glucan, an active component of Lentinus edodes consisting of β-1,3-glucans and β-1,6-glucans, was identified effective for the treatment of cancer. Since then, studies on pharmacologically active compounds from Lentinus edodes have been accelerated. Not only Lentinus edodes fruiting bodies, but also its spores, its mycelium and products isolated from cultivated fermentation broths have been studied in detail. These studies resulted in the discovery of several new active substances, such as lenthionine, lentinan, specific glycoproteins... . A detailed survey of the principal active compounds isolated from Lentinus edodes is given by Mizuno (1995). Recent studies on Lentinus edodes have demonstrated that the use of its extract results in an antitumor effect, antimicrobial properties, improved liver function and a reduction of viremia in patients with chronic hepatitis and an inhibition of human immunodeficiency virus infection in vitro (Hirasawa et al., 1999). A recent study revealed also that Lentinus edodes extracts strongly inhibit the formation of water-insoluble glucan from sucrose by the glycosyltransferase of streptococci (Shouji et al., 2000). In general, glycosyltransferases are involved in bacterial exopolysaccharide biosynthesis, which can occur in the intestinal tract and can favour bacterial adhesion.

Shittake mushrooms grow naturally on the dead wood of many hardwood species. In Japan, shiitake mushrooms have been cultivated on bed logs for 300 years, and today their cultivation is an important agricultural business. Because most Shiitake mushrooms have been cultivated on logs, many commercial varieties have been bred for log cultivation.

The cultivation of shiitake has remained in a primitive state until very recently. Shiitake traditionally have been grown on tree trunks, some types of trees supporting more abundant growth than others. Growth of shiitake on trees typically requires between one and two years until the first crop of fruiting bodies is produced.

US-5,123,203 describes a method for culture of fungi including shiitake (Lentinus edodes) using a method of sterilizing the substrate to allow cultivation of the desired fungi without contamination by competing organisms. The substrate is grain that is essentially cellulose free. Shiitake mushrooms have the ability to break down cellulose for essential nutrients, but can be more efficiently grown in a substrate containing these materials in an already usable form. Similarly, shiitake can break down lignin, which is a constituent of wood, but again shiitake can be cultivated more efficiently by providing the breakdown products instead of the lignin.

Further US-4 4,874,419 discloses a process for preparing a nutrient substrate composition suitable for growth of shiitake mushrooms consisting essentially of preparing a nitrogen-containing compost mixture consisting essentially of a vegetative waste, a mineral fertilizer and water, said mixture having a moisture content of about 65 to 80%; aerobically digesting the mixture at an ambient temperature range of between about 18 to 30°C. for between about 1 to 9 days; pasteurizing the aerobically digested mixture; and further digesting the pasteurized mixture at a mesothermic temperature of between about 45 to 55° C. for between about 3 to 5 days.

A possible way of treatment of the Shiitake is extraction. US-5,780,097 discloses a process for preparation of a powdery extract of shiitake mushroom comprising the steps of suspending a shiitake mushroom powder in water to form a suspension, subjecting the suspension to heat extraction at 80-90° C. for 30-60 minutes to obtain a hot water extract, concentrating the hot water extract by a factor of two to five, adding a cyclodextrin (CD) solution to the concentrated extract to afford a mixture, kneading the mixture under reduced pressure at 60.-90.°C. for 10-24 hours to obtain a CD clathrate and drying and pulverizing the clathrate.

Several antimicrobial substances have been isolated from Lentinus edodes, such as lentionine, disulfide analogues of lenthionine, lentinan and polyacetylene compounds (Hirasawa et al., 1999).

Nevertheless, in animal breeding, most attention is nowadays focussed on the use of the β-D-glucan fraction - isolated from Lentinus edodes or other fungi - as specific growth promoter (mainly via manipulation of probiotic content in the gastrointestinal tract) or as medicinal agent or nutraceutical for improvement of animal (and human) health (Patent WO 98/26787).

### Description of invention

In first instance, the present invention relates to the observation that providing to the animal the Lentinus edodes mushroom (or extracts or derivatives of it) as feed additive, changes the microbial ecosystem in the gastrointestinal tract of the animal in specific way resulting in a improved gastro-intestinal ecosystem.

The total amount of enteric pathogens are in a first stage surprisingly enumerated in the gastrointestinal tract i.e. a selective development of enteric pathogens within the gastrointestinal tract, and in a second stage, the enumerated enteric pathogens are very quickly excreted (for example within 2 weeks following application of Lentinus edodes) from the gastrointestinal tract of the animal.

This sequential mode of action is not earlier described for other types of growth promoters usable in animal breeding. Enteric pathogens can include following genera: Escherichia, Salmonella, Shigella, Klebsiella, Erwinia, Yersinia, Campylobacter, Helicobacter, Vibrio, Pseudomona as well as other Gramnegative bacteria.

By fast elimination of enteric pathogens from the gastrointestinal tract, in second instance, better performances, which are reflected in daily growth and feed conversion of the animal are obtained. The growth promoting effect of Lentinus edodes is already clearly visible at elution level of the enriched enteric pathogen strains from the gastrointestinal tract of the animal.

From these results and observations, it is expected that other fungi of genera within the Basidiomycetes are also suitable as growth promoter.

Other genera of mushrooms preferably include Ganoderma (Reishi), Cordyceps, Coriolus, Grifola (Maitake) and other fungi. A concentration up to about 0,01 to 20 weight% eventually combined with other raw materials or other growth promoting substances, such as antibiotics, probiotics, prebiotics, acids, etc.

In a preferred embodiment 0,5-5g and more preferably 1 g Lentinus edodes / 100g feed has been found to be particularly suitable.

In order that the present invention may be more clearly understood, the preferred form will be described with reference to the following examples.

### Example 1: Influence of Lentinus edodes on the microbial ecosystem in the gastrointestinal tract of poultry

3 x 40 one day old chickens were provided with following feeds: control feed, control feed supplemented with 3 ppm flavomycin and control feed supplemented with 1 % Lentinus edodes. Water and feed were supplied ad libitum. The chickens were contaminated at day 2 with caecum contents of 3 week old chickens (most critical period for gastrointestinal problems). At regular time intervals, chickens were dissected and the enteric pathogen contents in the small intestine were determined by plate counting on MacConkey agar.

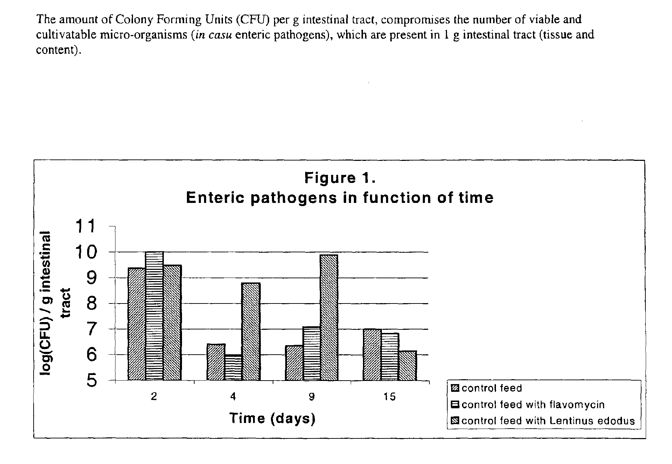

Figure 1 summarises the results.

From figure 1, it is clear that Lentinus edodes has during the first week a positive effect on enteric bacteria growth/survival. Only after one week, the enteric bacteria content in the intestinal tract lowers very quickly, even to a level lower then the control and flavomycin treatment.

Lentinus edodes (or its extracts or its derivatives) causes in first instance growth of enteric pathogens (growth favouring of enteric pathogens) followed by washout of the enumerated enteric pathogens strains. The use of Lentinus improves the microbial ecosystem in the gastrointestinal tract of the animal by specific enumeration of enteric pathogens followed by specific excretion, in order to improve weight gain, to reduce feed conversion and to improve this way the feed value and health and well-being of the animal.

The surprising effect of the invention is that Lentinus edodes or an other related fungi (or their extracts or derivatives) is usable as a specific growth promoter in animal breeding, by a specific sequential action, i.e. the enumeration of enteric pathogens in the gastrointestinal tract of the animal prior to wash-out of the enumerated enteric pathogens. As a result, less diarrhoea and better performances are obtained. Moreover, since enteric pathogens are excreted from the animal, healthier animals are obtained. Animals can include birds (poultry, ...) and mammalians (pigs, ruminants, pets, ...but also humans)

The observed effect is obtained during normal transit of the (eventually dried) fungus (or an extract or a derivative) through the gastrointestinal tract of the animal.

### Example 2: Influence of Lentinus edodes on chicken performance

The same experimental conditions were applied as described in experiment 1. In this example, daily growth and feed conversion were monitored after 13 days. The results are summarised in table 1.

**Table 1.**

| Influence of Lentinus edodes on chicken performance | | | |
|---|---|---|---|
| | Control | Control +flavomycin | Control+Lentinus edodes |
| Weight/chicken at day 1 | 43.1 | 43.54 | 43.66 |
| Weight/chicken at day 13 | 164.1 | 208.8 | 211.5 |
| Feed conversion/chicken | 1.80 | 1.51 | 1.48 |

From table 1, it can be concluded that use of Lentinus edodes in this particular test gave similar results as those obtained with a traditional growth promoter (flavomycin). Promoting the growth of animals is herein defined as promoting growth in terms of weight gain in time (growth rate) and/or promoting growth in terms of feed efficiency (feed conversion ratio). Nevertheless, the mode of action is not comparable (see example 1). The use of a Basidiomycetes according to the invention is in particular useful for poultry and mammals, such as pigs.

In a fifth aspect these preparations according to the claims1-3 is useful as a medicine, in particular for the treatment of diarrhoea.

### References

AARESTRUP, F.M. (1999). Association between the consumption of antimicrobial agents in animal husbandry and the occurrence of resistant bacteria among food animals. International Journal of Antimicrobial Agents, 12, 279-285.
BARTON, M.D. (1998). Does the use of antibiotics in animals affect human health. Aust. Vet. J., 76, 177-180.
BOWER, C.K., DAESCHEL, M.A. AND MCGUIRE, J. (1998). Protein antimicrobial barriers to bacterial adhesion. J. Dairy Sci., 81, 2771-2778.
BROWN, I.L., WANG, X., TOPPING, D.L., PLAYNE, M.J. AND CONWAY, P.L. (1998). High amylose maize starch as a versatile prebiotic for use with probiotic bacteria. Food, 50, 603-610.
CHANG, S.T. (1993). Mushroom Biology: the impact on mushroom production and mushroom products. In: CHANG, S.T., BUSWELL, J.A., CHIU, S.W. (eds.). Mushroom biology and mushroom products. Hong Kong, Chinese University Press, 3-20.
CHIQUETTE, J. AND BANCHAAR, C. (1998). Effect of diet and probiotic addition on chemical composition of free and particle associated bacterial populations of the rumen. Can. J. Anim. Sci., 78, 115-120.
CHIRICA, L.C., GURAY, T., GURAKAN, G.C. AND BOZOGLU, T.F. (1998). Characterisation of extracellular beta-lactamase from penicillin G- resistant cell of Streptococcus thermophilus. J. Food Prot., 61, 896-898.
DE KONING, W. AND HONGBIAO, D. (1999). Chinese herbs as feed additives. Feed Mix, 6(3), 17-18.
DUPONT, H.L. AND STEELE, J.H. (1987). Use of antimicrobial agents in animal feeds: implications for human health. Rev. Infect. Dis., 9, 447-460.
ECKEL, B. (1997). Feed acids in piglet feeding. Feed magazine, 1, 28-31.
ECKEL, B. (1999). Probiotics can improve intestinal microbe balance and feed hygiene. Feed Tech, 3(7), 39-41.
FINLAND, M. (1971). Changes in susceptibility of selected pathogenic bacteria to widely used antibiotics. Ann. NY Acad. Sci., 182, 5-20.
GARRIGA, M., PASCUAL, M., MONFORT, J.M. AND HUGAS, M. (1998). Selection of lactobacilli for chicken probiotic adjuncts. J. Appl. Microbiol., 84, 125-132.
GUILLOT, J.F. (1989). Apparition et évolution de la résistance bactérienne aux antibiotiques. Ann. Rech. Vet., 20, 3-16.
HAMILTON, J.O.C. (1994). Who will stop the mutant microbes. Business Week, August 1, 52-53.
HEDGES, R.W. AND JACOB, A.E. (1974). Transposition of ampicillin resistance from RP4 to other replicons. Mol. Gen. Genet., 132, 31-40.
HIRASAWA, M., SHOUJI, N., NETA, T., FUKUSHIMA, K. AND TAKADA, K. (1999). Three kinds of antibacterial substances from Lentinus edodes (Berk.) Sing. (Shiitake, an edible mushroom). International Journal of Antimicrobial Agents, 11, 151-157.
HOUDIJK, J.G.M., BOSCH, M.W., TAMMINGA, S., VERSTEGEN, M.W.A., BERENPAS, E.B. AND KNOOP, H. (1999). Apparent Ileal and Total-Tract nutrient digestion by pigs as affected by dietary nondigestible oligosaccharides. J. Anim. Sci., 77, 148-158.
IJI, P.A. AND TIVEY, D.R. (1998). Natural and synthetic oligosaccharides in broiler chicken diets. World's Poultry Science Journal, 54, 129-143.
LIANG, C. (1997). Organic acids control harmful micro-organisms in poultry feed. Zootehnica International, January, 36-39.
MAZZA, G. (ed.) (1998). Functional Foods. Technomic Publishing Company, Pennsylvania.
MIZUNO, T. (1995). Shiitake, Lentinus edodes: functional properties for medicinal and food purposes. Food Reviews International, 11(1), 111-128.
MUIRHEAD, S. (1998). EU ban of antibiotics draws sharp criticism. Feedstuffs, 70, 1-4.
NEU, C.H., , RICHMOND, M., MITSUHASHI, S., NORD, C.E., ROSS, G.W., KNOWLES, J.R. AND SUTHERLAND, R. (1980). Beta-lactamase: a major form of bacterial resistance. In: Current chemotherapy and infectious diseases. Springer Verlag, Berlin, 19-25.
OLSEN, R. (1996). Experience with mannanoligosaccharides in commercial turkey production. Zootechnica International, August, 38-39.
PATENT WO/98/26787. Prebiotics and probiotics.
PRESCOTT, J.F. (1997). Antibiotics: miracle drugs or pig food. Can. Vet. *J.,* 38, 763-766.
RADECKI, S.V., JUHL, M.R. AND MILLER, E.R. (1988). Fumaric and citric acids as feed additives in starter pig diets: effect on performance and nutrient balance. J. Anim. Sci., 66, 2598-2605.
ROSS, I.W. (1999). Antibiotics in animal feed stocks and implications of the European Commission ban. Food Test Anal., 5, 8-10.
SHOUJI, N., TAKADA, K., FUKUSHIMA, K. AND HIRASAWA, M. (2000). Anticaries effect of a component from Shiitake (an edible mushroom). Caries Research, 34(1), 94-98.
TANNOCK, G.W. (ed.) (1999). Probiotics: a critical review. Horizon Scientific Press, Wymondham.
THOMPSON, R. (1986). R plasmid transfer. J. Antimicrob. Chemother., 18, 13-23.

## Claims

1. A growth promoter suitable for an animal consisting essentially of a fungi, a extract, a derivative or a mixture thereof, wherein said fungi is chosen from a genera within the Basidiomycetes.

2. A growth promoter according to claim 1 wherein the fungi is chosen from the genera belonging to the order Agaricales or Aphyllopharales of Homobasidiae, such as for example Armillariella mellea (Fr.) Karst, Tricholoma matsutake (S. Ito et Imai) Sing., Lentinus edodes, (Berk.) Sing.or Shiitake, Coriolus versicolor (Fr.) Quel., Grifola gigantea (Fr.) Pilat, Favolus Arcularius (Fr.) Ames, Ganoderma (Reishi), Ganoderma (Reishi), Cordyceps, Coriolus or Grifola (Maitake).

3. A growth promoter according to claim 2 wherein the fungi is Lentinus edodes, (Berk.) Sing.(Shiitake).

4. An animal feed comprising 0,01 to 20 weight% of a growth promoter as claimed in any of the claims 1-3.

5. Use of an animal feed according to claim 4 as poultry or pig feed, in particular in their early lifestage.

6. A method for the improvement of weight gain and/or for reducing the feed conversion and/or for the improvement of the feed value and/or health and well-being of the animal by providing animals with a fungi or an extract, derivative or a mixture thereof as claimed in claims 1-3 resulting in a sequential action of the specific enumeration of enteric pathogens prior to their specific excretion.

7. The method according to claim 6, wherein different genera of fungi (mushrooms) is applicable, such as Lentinus (Shiitake), Ganoderma (Reishi), Grifola (Maitake) and other fungi, either alone or in combination which each other.

8. The method according to claim 7, wherein the fungus is Lentinus edodes.

9. The method according to any of the claims 6, 7 or 8 wherein the observed effect is obtained during normal transit and digestion process of the fungus or its extract or its derivative or a mixture thereof, through the gastrointestinal tract of the animal.

10. The method according to any of the claims 6-9, comprising spores, mycelium and products isolated from cultivated fermentation broths are used, either alone or in combination.

11. The method according to any of the claims 6-10, wherein the fungus concentration or an extract, derivative or a mixture thereof are used of up to about 0.01-20 weight%.

12. The method according to any of the claims 6-11, wherein fungus chosen from the group comprising antibiotics, probiotics, prebiotics or acids.

13. The method according to any of the claims 6-12, wherein the fungus or an extract, derivative or mixture thereof is dosed on dry basis from 0,5-5 g / 100 g feed.

14. The method according to any of the claims 6-13, wherein the enteric pathogens include the genera Escherichia, Salmonella, Shigella, Klebsiella, Erwinia, Yersinia, Campylobacter, Helicobacter, Vibrio, Pseudomona as well as other Gram negative bacteria.

15. The method according to any of the claims 6-14, wherein the animal is classified as poultry.

16. The method according to any of the claims 6-14, wherein the animal is classified as a mammal, in particular a pig.

17. Use of a growth promoter according to any of the claims 1-3, for use as a medicine in the treatment of diarrhoea.
